# EUROPEAN PATENT APPLICATION

(11) **EP 2 123 259 A1**
(43) Date of publication of application: **25.11.2009**
(21) Application number: 07832933.1
(22) Date of filing: 03.12.2007
(51) Int. Cl.: A61K 9/127, A61K 38/44, A61K 47/24, A61K 47/28, A61P 17/18

(54) **LIPOSOME PREPARATION FOR IONTOPHORESIS HAVING ANTIOXIDANT COMPONENT ENCAPSULATED THEREIN**

(30) Priority: 16.01.2007 JP 2007007130
(71) Applicant: Hokkaido University, Kita-ku Sapporo-shi Hokkaido 060-0808 (JP); TTI ellebeau, Inc., Tokyo 140-0002 (JP)
(72) Inventor: KOGURE, Kentaro, Sapporo-shi Hokkaido 060-0812 (JP); MIYASHITA, Moeko, Sapporo-shi Hokkaido 060-0812 (JP); HARASHIMA, Hideyoshi, Sapporo-shi Hokkaido 060-0812 (JP)
(74) Representative: Liesegang, Eva
(86) International application number: PCT/JP2007/073313
(87) International publication number: WO 2008/087803

(57) **Abstract**

The present invention relates to a liposome formulation for iontophoresis for administering an antioxidant component to a living organism by iontophoresis, the liposome formulation being **characterized in that** the antioxidant component is encapsulated in a liposome.

## Description

### Background of the Invention

### Field of the Invention

The present invention relates to a technology for transdermally administering an antioxidant component by iontophoresis, in particular, a liposome formulation for iontophoresis which: enables the stable, efficient delivery of an antioxidant component to each of the deep part of a pore and an intradermal tissue around the pore by iontophoresis; and is particularly useful for the prevention or therapy of a dermatopathy resulting from ultraviolet light.

### Background Art

An influence of an increase in quantity of ultraviolet light due to, for example, the destruction of an ozone layer on a human body has been of concern in recent years. In particular, a dermatopathy resulting from ultraviolet light is a serious problem.

Such dermatopathy due to ultraviolet light is known to occur not only on the surface of a skin but also in the skin. Therefore, the prevention or therapy of, in particular, a dermatopathy due to ultraviolet light that permeates even into a skin, such as UVA requires the effective inhibition of the production of active oxygen in the skin or the quick removal of active oxygen which has been once produced.
However, it has been difficult to remove such active oxygen that is generated in a skin by a conventional, ordinary technology.

By the way, a method of introducing an ionic drug placed on the surface of a skin or mucosa (hereinafter simply referred to as "skin") at a predetermined site of a living organism into the body of the living organism through the skin (causing the drug to permeate into the body) by applying an electromotive force to the skin for driving the drug is referred to as iontophoresis (see JP 63-35266 A or the like).

For example, an ion having positive charge is driven (transported) into a skin on the anode side of the electrical system of an iontophoresis device. On the other hand, an ion having negative charge is driven (transported) into the skin on the cathode side of the electrical system of the iontophoresis device. According to iontophoresis, the first-pass effect of a liver can be avoided, and the initiation and interruption of the administration of a drug or the like can be simply controlled. In view of the foregoing, in recent years, iontophoresis has been attracting attention because of its potential to serve as an administration method intended for not only a local action but also a systemic action.

In iontophoresis, a drug is generally administered to a living organism mainly through the corneal layer of the skin of the living organism. However, the corneal layer is a fat-soluble, high-density layer, and it is often difficult to cause a substance having high water solubility or a polymer such as a peptide or a nucleic acid to penetrate through the layer. In addition, a substance having no charge involves the following problem: iontophoresis cannot be applied to the substance as it is.

In order that a substance having various physical and chemical properties may be transdermally administered, investigation has been conducted on the application of a chargeable liposome as a carrier in iontophoresis (Median VM et al., International Journal of Pharmaceutics, Dec. 8, 2005: 306 (1-2): 1-14. Epub Nov. 2, 2005 Epub 2005 Nov 2. Review). However, the liposome has a large particle diameter, and it is difficult for the liposome to penetrate through a corneal layer as it is.

Meanwhile, it has been know that a pore penetrating from the surface of a skin to the deep part of the skin is used as a target for the efficient transdermal administration of a liposome (see, for example, Hoffman RT et al., Nat Med. 1995 Jul; 1(7): 705-706; and Fleisher D et al, Life Sci. 1995; 57(13): 1293-1297).

Further, in recent years, investigation has been conducted on the administration of a liposome through a pore also in iontophoresis. For example, Protopapa EE et al. have reported that they delivered a liposome in which an enzyme had been encapsulated to a hair-matrix stem cell in a pore by iontophoresis (Protopapa EE et al., J Eur Acad Dermatol Venereol. 1999 Jul; 13(1): 28-35).

In addition, Han I et al. have reported that they delivered a liposome in which 5-aminolevulinic acid as a drug for photo-dynamic therapy had been encapsulated to, for example, a pore sebaceous crypt in the upper portion of a pore by iontophoresis (Han I et al., Arch Dermatol Res. 2005 Nov; 295(5): 210-217. Epub 2005 Nov 11). Further, Han I et al. have reported that they administered a liposome in which adriamycin as a therapeutic agent for a pore-related tumor had been encapsulated to a pore by iontophoresis (Han I et al., Exp Dermatol. 2004 Feb; 13(2): 86-92).

The main purpose of conventional iontophoresis using a pore as a target is as follows: a skin surface disease or the like is defined as an object to be cured, and a drug is delivered to the surface layer portion of a skin tissue. However, in case of the administration of a drug or the like intended for a systemic action in iontophoresis, the delivery of the drug to a systemic circulation system through a subcutaneous blood vessel present in the deep part of a pore has been demanded.

Further, the therapy or prevention of such dermatopathy resulting from ultraviolet light that permeates even into a skin as described above requires the reliable delivery of an antioxidant component to an intradermal tissue around a pore for the inhibition of the production of active oxygen in the skin or the quick removal of produced active oxygen. Therefore, the stable, efficient delivery of an antioxidant component to each of the deep part of a pore and an intradermal tissue around the pore is important in iontophoresis using the pore as a target.

### Summary of the Invention

Therefore, an object of the present invention is to provide a formulation for iontophoresis that enables the stable, efficient delivery of an antioxidant component to each of the deep part of a pore and an intradermal tissue around the pore in iontophoresis.

The inventors of the present invention have found that the application of a liposome in which an antioxidant component has been encapsulated to iontophoresis enables the stable, efficient delivery of the antioxidant component to each of the deep part of a pore and an intradermal tissue around the pore.

The liposome formulation for iontophoresis according to the present invention is for administering an antioxidant component to a living organism by iontophoresis and **characterized in that** the antioxidant component is encapsulated in a liposome.

According to a preferred aspect of the present invention, the antioxidant component is an antioxidant enzyme, and more specifically, the antioxidant enzyme is superoxide dismutase (SOD).

According to another aspect of the present invention, the antioxidant enzyme may be glutathione peroxidase (GSD-Px) and/or catalase. In addition, the liposome formulation for iontophoresis is composed of a combination of liposomes in each of which superoxide dismutase (SOD), glutathione peroxidase (GSH-Px), or catalase is encapsulated as the antioxidant component.

According to a preferred aspect of the present invention, the liposome contains, as constituents, a cationic lipid, and an amphiphilic glycerophospholipid containing both a saturated fatty acid and an unsaturated fatty acid as constituent fatty acids.

Further, the present invention includes: the liposome formulation used for prevention or therapy of a dermatopathy resulting from ultraviolet light; an electrode assembly for administering an antioxidant component to a living organism by iontophoresis; and an iontophoresis device including the electrode assembly.

Because the present invention is composed of a formulation containing a liposome which is stable and can move in a pore, and in which an antioxidant component is encapsulated, the liposome formulation can be delivered to the inside of a skin by iontophoresis. As a result, active oxygen produced in the skin can be extinguished, so the present invention is considerably useful for the prevention, reduction, and therapy of a dermatopathy resulting from, for example, irradiation with ultraviolet light. In addition, the present invention exerts the following excellent effect: injuries resulting from the generation of active oxygen in a skin which have been conventionally considered to be hard to prevent can be prevented, whereby not only the prevention of a dermatopathy including a skin inflammation but also the suppression of the generation of a spot and a wrinkle resulting from the injuries can be achieved.

### Brief Description of the Drawings

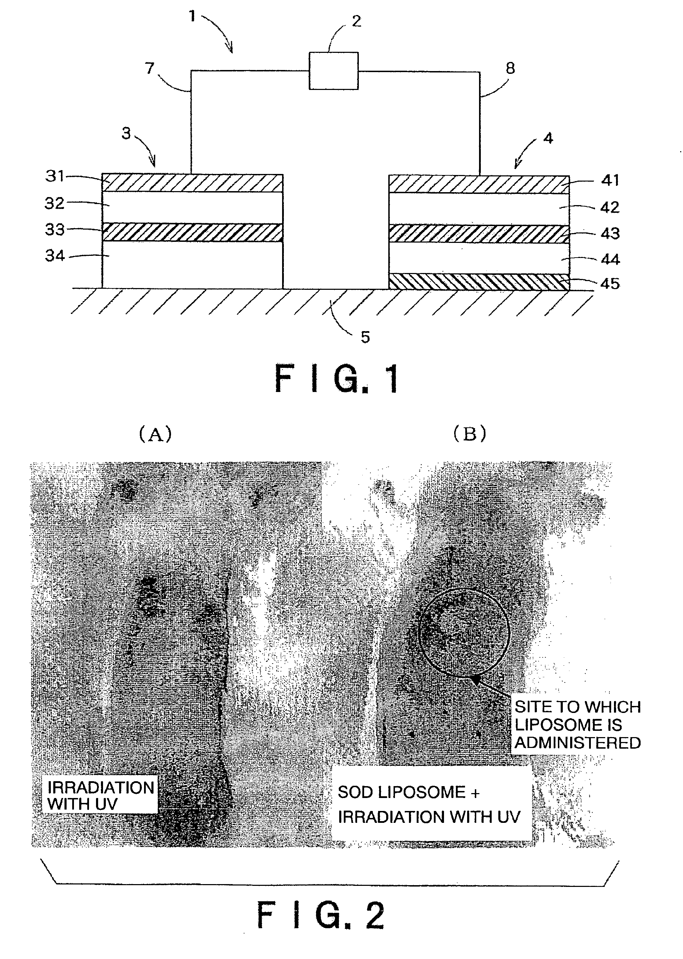

Fig. 1 is a view showing the outline of an iontophoresis device used in an in vivo skin penetration test upon administration of a liposome formulation according to the present invention.
Figs. 2(A) and 2(B) are each a photograph showing the biological morphology of the skin of each of a rat to which an SOD-encapsulated liposome formulation is not administered (A: irradiation with UV) and a rat to which the SOD-encapsulated liposome formulation is administered (B: SOD liposome + irradiation with UV) after the irradiation with UV.

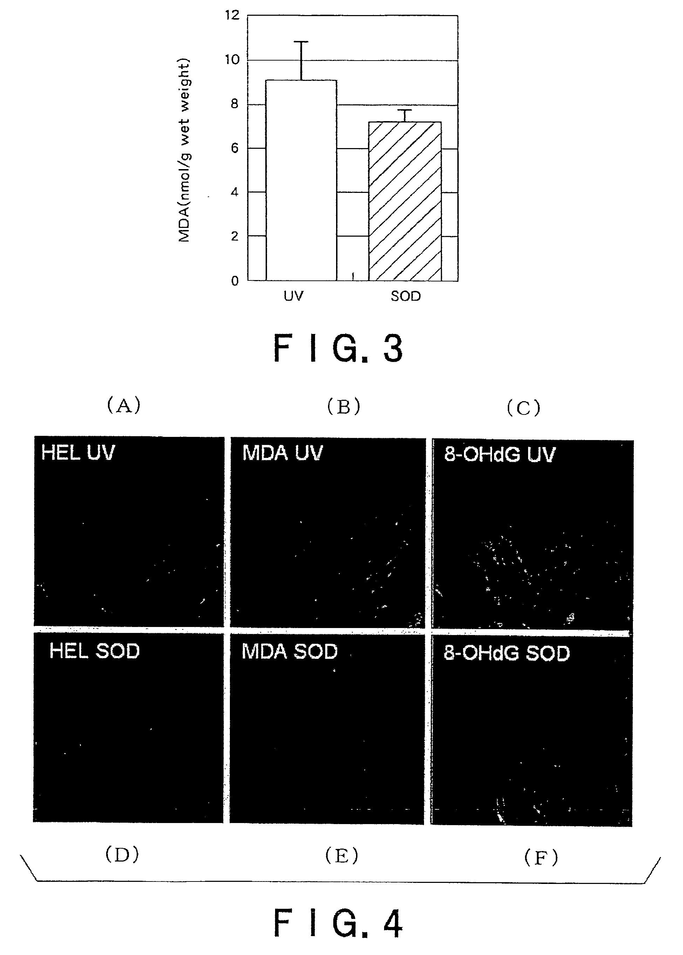

Fig. 3 is a graph showing the result of the determination of the amount of a lipid peroxide (malon dialdehyde: MDA) of each of the skin of a rat to which the SOD-encapsulated liposome formulation is not administered (UV) and the skin of a rat to which the SOD-encapsulated liposome formulation is administered (SOD) after irradiation with UV.
Figs. 4(A) to 4(F) are each a micrograph of a skin section of each of a rat to which the SOD-encapsulated liposome formulation is not administered (UV) and a rat to which the SOD-encapsulated liposome formulation is administered (SOD) after irradiation with UV, the section being subjected to immunostaining with each of various peroxidative damage marker antibodies.

### Specific Description of the Invention

In the specification of the present invention, the term "C" in a group or in part of a group refers to "the total number of carbon atoms" in the group or in part of the group unless otherwise stated. Therefore, for example, the term "C1 to C6 saturated fatty acid" refers to a "saturated fatty acid having a total of 1 to 6 carbon atoms", and the term "C12 to 31 fatty acid cholesteryl" refers to a fatty acid cholesteryl having a "fatty acid having a total of 12 to 31 carbon atoms".

In addition, the term "alkyl", "alkenyl", or "alkynyl" as a group or as part of a group means a straight chain, branched, or cyclic alkyl, alkenyl, or alkynyl group; the group is preferably straight chain or branched, or more preferably straight chain.

In addition, a fatty acid in a "fatty acid cholesteryl" or a "fatty acid dihydrocholesteryl" may be saturated or unsaturated. Further, the above fatty acid may be a straight chain, branched, or cyclic fatty acid. In addition, a fatty acid in a "fatty acid cholesteryl" is preferably straight chain, and a fatty acid in a "fatty acid dihydrocholesteryl" is preferably straight chain.

In addition, the term "aryl" refers to a phenyl or naphthyl group unless otherwise stated, and the term "heteroaryl" refers to a five- or six-membered heteroaryl group (five- or six-membered aromatic heterocyclic group) containing 1 to 3 nitrogen, oxygen, or sulfur atoms unless otherwise stated.

The term "front surface" refers to a side close to the skin of a living organism on the path of a current flowing in an electrode assembly upon administration of a liposome.

### Liposome for iontophoresis

As described above, a formulation according to the present invention contains an antioxidant component encapsulated in a liposome.
In a preferred aspect of the present invention, a liposome in which an antioxidant component should be encapsulated contains, as constituents, a cationic lipid, and an amphiphilic glycerophospholipid containing both a saturated fatty acid and an unsaturated fatty acid as constituent fatty acids. It is a surprising fact that such liposome containing certain constituents is advantageous for the stable delivery of the antioxidant component to each of the deep part of a pore and an intradermal tissue around the pore.

In addition, the cation lipid as a constituent of the liposome in the present invention is preferably a C1 to C20 alkane substituted by a C1 to C20 acyloxy group and a tri(C1 to C4)alkylammonium group.

Further, the above C1 to C20 alkane is preferably a C1 to C5 alkane, or more preferably a C1 to C3 alkane.

In addition, the number of the C1 to C20 acyloxy group as a substituent in the above C1 to C20 alkane is preferably 1 to 4, or more preferably 2. In addition, the above C1 to C22 acyloxy group is preferably a C1 to C20 acyloxy group, or more preferably a C1 to C18 acyloxy group.

In addition, specific examples of the above C1 to C22 acyloxy group include an alkylcarbonyloxy group, an alkenylcarbonyloxy group, an alkynylcarbonyloxy group, an arylcarbonyloxy group, and a heteroarylcarbonyloxy group. Of those, an alkylcarbonyloxy group, an alkenylcarbonyloxy group, or an alkynylcarbonyloxy group is preferable, and an alkenylcarbonyloxy group is more preferable.

In addition, the number of the tri(C1 to C6)alkylammonium group as a substituent in the above C1 to C20 alkane is preferably 1 to 4, or more preferably 1. In addition, the above tri(C1 to C6)alkylammonium group is preferably a tri(C1 to C4)alkylammonium group. Although a counter ion for the above trialkylammonium group is not particularly limited, examples of the counter ion include a chlorine ion, a bromine ion, an iodine ion, a fluorine ion, a sulfite ion, and a nitrite ion. Of those, a chlorine ion, a bromine ion, and an iodine ion are preferable.

More specifically, as the cation lipid, 1,2-dioleoyloxy-3-trimethylammonium propane (DOTP), dioctadecyldimethylammonium chloride (DODAC), N-(2,3-dioleyloxy) propyl-N,N,N-trimethylammonium (DOTMA), didodecylammonium bromide (DDAB), 1,2-dimyristoyloxypropyl-3-dimethylhydroxyethyl ammonium (DMRIE), and 2,3-dioleoyloxy-N-[2(sperminecarboxamido ) ethyl]-N,N-dimethyl-1-propanaminum trifluoroacetate (DOSPA) are preferably mentioned. DOTAP is more preferred.

In addition, the amphiphilic glycerophospholipid in the present invention is characterized by including both a saturated fatty acid and an unsaturated fatty acid as consituent fatty acids. Examples of the amphiphilic glycerophospholipid include phosphatidylcholine, phosphatidyl ethanolamine, phosphatidyl glycerol, phosphatidic acid, cardiolipin, phosphatidyl serine, and phosphatidyl inositol. Preferred is phosphatidylcholine, and more preferred is a yolk phosphatidylcholine.

Among the consituent fatty acids of the amphiphilic glycerophospholipid, a C12 to C22 saturated fatty acid is preferred and a C14 to C18 saturated fatty acid is more preferred as the saturated fatty acid. Specific examples of the saturated fatty acid is at least one memeber selected from the group consisting of palmitic acid, lauric acid, myristic acid, pentadecylic acid, margaric acid, stearic acid, tuberculostearic acid, arachidic acid, and behenic acid. More preferred is palmitic acid, myristic acid, pentadecylic acid, margaric acid, or stearic acid.

In addition, the unsaturated fatty acid out of the constituent fatty acids of the amphiphilic glycerophospholipid is preferably a C14 to C22 unsaturated fatty acid, or more preferably a C14 to C20 unsaturated fatty acid. Further, the above unsaturated fatty acid contains preferably 1 to 6, or more preferably 1 to 4 carbon-carbon double bonds.

Specific examples of the unsaturated fatty acid is preferably at least one member selected from the group consisting of oleic acid, myristoleic acid, palmitoleic acid, elaidic acid, vaccenic acid, gadoleic acid, erucic acid, nervonic acid, linoleic acid, α-linoleic acid, eleostearic acid, stearidonic acid, arachidonic acid, eicosapentaenoic acid, clupanodonic acid, and docosahexaenoic acid. More preferred is oleic acid, myristoleic acid, palmitoleic acid, elaidic acid, vaccenic acid, gadoleic acid, erucic acid, nervonic acid, linoleic acid, α-linoleic acid, eleostearic acid, or stearidonic acid.

In addition, according to another preferred aspect of the present invention, the saturated fatty acid is at least one member selected from the group consisting of palmitic acid, myristic acid, pentadecylic acid, margaric acid, and stearic acid, and the unsaturated fatty acid is at least one member selected from the group consisting of oleic acid, myristoleic acid, palmitoleic acid, elaidic acid, vaccenic acid, gadoleic acid, erucic acid, nervonic acid, linoleic acid, α-linoleic acid, eleostearic acid, stearidonic acid, and arachidonic acid in the amphiphilic glycerophospholipid.

According to a more preferred aspect of the present invention, the liposome further contains sterols as a constituent. The sterols in the present invention are preferably at least one selected from the group consisting of cholesterol, a C12 to C31 fatty acid cholesteryl, a C12 to C31 fatty acid dihydrocholesteryl, polyoxyethylene cholesteryl ether, and polyoxyethylene dihydrocholesteryl ether, more preferably at least one selected from the group consisting of cholesterol, cholesteryl lanoate, cholesteryl oleate, cholesteryl nonanoate, macadamia nut fatty acid cholesteryl, and dihydrocholesterol polyethylene glycol ether (specifically, for example, dihydrocholes-30), or still more preferably cholesterol.

### Combinations of constituents

As described above, the liposome of the present invention preferably contains, as constituents, all of the cationic lipid, the sterols, and the amphiphilic glycerophospholipid containing, as constituent fatty acids, both a saturated fatty acid and an unsaturated fatty acid.

According to a more preferred aspect of the present invention, the constituents of the liposome contain: a C1 to C20 alkane obtained by being substituted by two C1 to C22 acyloxy groups and one tri(C1 to C6)alkylammonium group; sterols; and an amphiphilic glycerophospholipid containing, as constituent fatty acids, both a C12 to C22 saturated fatty acid and a C14 to C22 unsaturated fatty acid having 1 to 6 carbon-carbon unsaturated double bonds.

In addition, according to a still more preferred aspect of the present invention, the constituents of the liposome contain: 1,2-dioleoyloxy-3-trimethylammonium propane; at least one sterol selected from the group consisting of cholesterol, a C12 to C31 fatty acid cholesteryl, a C12 to C31 fatty acid dihydrocholesteryl, polyoxyethylene cholesteryl ether, and polyoxyethylene dihydrocholesteryl ether; and an amphiphilic glycerophospholipid containing, as constituent fatty acids, both a C12 to C22 saturated fatty acid and a C14 to C22 unsaturated fatty acid having 1 to 6 carbon-carbon unsaturated double bonds.

In addition, according to a still more preferred aspect of the present invention, the constituents of the liposome contain: 1,2-dioleoyloxy-3-(trimethylammonium)propane; at least one sterol selected from the group consisting of cholesterol, cholesteryl lanolate, cholesteryl oleate, cholesteryl nonanoate, macadamia nut fatty acid cholesteryl, and polyoxyethylene dihydrocholesteryl ether; and an amphiphilic glycerophospholipid containing, as constituent fatty acids, both a C12 to C22 saturated fatty acid and a C14 to C22 unsaturated fatty acid having 1 to 6 carbon-carbon unsaturated double bonds.

In addition, according to a still more preferred aspect of the present invention, the constituents of the liposome contain: 1,2-dioleoyloxy-3-(trimethylammonium)propane; sterols; and an amphiphilic glycerophospholipid containing, as constituent fatty acids, both of at least one saturated fatty acid selected from the group consisting of palmitic acid, lauric acid, myristic acid, pentadecylic acid, margaric acid, stearic acid, tuberculostearic acid, arachidic acid, and behenic acid, and at least one unsaturated fatty acid selected from the group consisting of oleic acid, myristoleic acid, palmitoleic acid, elaidic acid, vaccenic acid, gadoleic acid, erucic acid, nervonic acid, linoleic acid, α-linolenic acid, eleostearic acid, stearidonic acid, arachidonic acid, eicosapentaenoic acid, clupanodonic acid, and docosahexanoic acid.

In addition, according to a still more preferred aspect of the present invention, the constituents of the liposome contain: 1,2-dioleoyloxy-3-(trimethylammonium)propane; at least one sterol selected from the group consisting of cholesterol, a C12 to C31 fatty acid cholesteryl, a C12 to C31 fatty acid dihydrocholesteryl, polyoxyethylene cholesteryl ether, and polyoxyethylene dihydrocholesteryl ether; and an amphiphilic glycerophospholipid containing, as constituent fatty acids, both of at least one saturated fatty acid selected from the group consisting of palmitic acid, lauric acid, myristic acid, pentadecylic acid, margaric acid, stearic acid, tuberculostearic acid, arachidic acid, and behenic acid, and at least one unsaturated fatty acid selected from the group consisting of oleic acid, myristoleic acid, palmitoleic acid, elaidic acid, vaccenic acid, gadoleic acid, erucic acid, nervonic acid, linoleic acid, α-linolenic acid, eleostearic acid, stearidonic acid, arachidonic acid, eicosapentaenoic acid, clupanodonic acid, and docosahexanoic acid.

In addition, according to a still more preferred aspect of the present invention, the constituents of the liposome contain: 1,2-dioleoyloxy-3-trimethylammonium propane; at least one sterol selected from the group consisting of cholesterol, cholesteryl lanolate, cholesteryl oleate, cholesteryl nonanoate, macadamia nut fatty acid cholesteryl, and polyoxyethylene dihydrocholesteryl ether; and an amphiphilic glycerophospholipid containing, as constituent fatty acids, both of at least one saturated fatty acid selected from the group consisting of palmitic acid, lauric acid, myristic acid, pentadecylic acid, margaric acid, stearic acid, tuberculostearic acid, arachidic acid, and behenic acid, and at least one unsaturated fatty acid selected from the group consisting of oleic acid, myristoleic acid, palmitoleic acid, elaidic acid, vaccenic acid, gadoleic acid, erucic acid, nervonic acid, linoleic acid, α-linolenic acid, eleostearic acid, stearidonic acid, arachidonic acid, eicosapentaenoic acid, clupanodonic acid, and docosahexanoic acid.

In addition, according to a still more preferred aspect of the present invention, the amphiphilic glycerophospholipid is phosphatidylcholine.
In addition, according to a still more preferred aspect of the present invention, the constituents of the liposome contain 1,2-dioleoyloxy-3-(trimethylammonium)propane, cholesterol, and a yolk phosphatidylcholine.

In addition, a molar ratio between the cationic lipid and the amphiphilic glycerophospholipid in the liposome in the present invention is preferably 3 : 7 to 7 : 3, or more preferably 4 : 6 to 6 : 4 in consideration of the stability and delivery efficiency of the liposome in iontophoresis. In addition, when the liposome contains the sterols, a molar ratio between the cationic lipid and the sterols is preferably 3 : 7 to 7 : 3, or more preferably 4 : 6 to 6 : 4. In addition, a molar ratio between the amphiphilic glycerophospholipid and the sterols in the liposome in the present invention is preferably 3 : 7 to 7 : 3, or more preferably 4 : 6 to 6 : 4. In addition, a molar ratio between the cationic lipid, and a sum of the amphiphilic glycerophospholipid and the sterols in the liposome in the present invention is preferably 3 : 7 to 7 : 3, or more preferably 4 : 6 to 6 : 4. In addition, according to an aspect of the present invention, a molar ratio between the cationic lipid, the amphiphilic glycerophospholipid, and the sterols is about 2 : 1 : 1.

In addition, the liposome in the present invention has an average particle diameter of preferably about 400 nm or more, or more preferably 400 to 1,000 nm. The average particle diameter of the liposome can be identified by a method such as dynamic light scattering, static light scattering, observation with an electron microscope, or observation with an atomic force microscope.

### Antioxidant component

Although the antioxidant component to be encapsulated in the present invention is not particularly limited as long as the antioxidant component can be encapsulated in the liposome, the antioxidant component is preferably an antioxidant enzyme such as an active oxygen-extinguishing enzyme.

To be more specific, a preferable antioxidant enzyme is superoxide dismutase (SOD) as an active oxygen-extinguishing enzyme. Further, glutathione peroxidase (GSH-Px) and catalase can also be used.

Further, in the present invention, a liposome formulation may be composed of a combination of liposomes in each of which superoxide dismutase (SOD), glutathione peroxidase (GSH-Px), or catalase is encapsulated as an antioxidant component.

Further, in the present invention, in addition to the foregoing, for example, a fat-soluble antioxidant compound such as α-tocopherol (vitamin E), β-carotene, astaxanthin, lycopene, or capsaicin, or a water-soluble antioxidant compound such as ascorbic acid (vitamin C), a polyphenol such as curcumin, or cysteine can be used as the antioxidant component.

### Production method

For example, the following method can be employed as a method of producing each of the liposome in the present invention and a composition containing the liposome in the present invention. First, a cationic lipid, an amphiphilic glycerophospholipid, and, as desired, sterols are mixed in an organic solvent such as CHCl₃ at a desired ratio, whereby a suspension is obtained. After the solvent of the suspension has been removed by distillation under reduced pressure, the addition, and removal by distillation under reduced pressure, of the organic solvent are repeated, whereby a lipid film is obtained. Next, 10 to 50 mM of a buffer such as 2-[4-(2-hydroxyethyl)-1-piperazinyl]ethanesulfonic acid (HEPES) and a desired amount of an antioxidant component are added to the lipid film. The resultant mixed liquid is left at room temperature for 10 minutes so as to be hydrated, and is then subjected to sonication. The sonication is performed under, for example, the following conditions, but the conditions for the sonication are not limited to the following conditions: 85 W, room temperature, and about 1 minute. Further, the mixed liquid is treated with, for example, a membrane filter or an extruder so as to have an adjusted particle diameter, whereby the liposome in the present invention is obtained. Further, the resultant liposome is further appropriately mixed with, for example, a pharmaceutically acceptable carrier, whereby the liposome formulation according to the present invention can be obtained.

Although the pharmaceutically acceptable carrier to be used in the present invention is not particularly limited as long as the carrier does not inhibit the administration of the liposome by iontophoresis, examples of the carrier include additives such as a surfactant, a lubricant, a dispersant, a buffer such as HEPES, a preservative, a solubilizing agent, an antiseptic, a stabilizer, an antioxidant, and a colorant. Further, the liposome formulation according to the present invention can be turned into a proper formulation as desired as long as the formulation does not inhibit the administration of the liposome by iontophoresis. However, the formulation is preferably turned into a solution or a suspension together with an HEPES buffer or an electrolyte solution to be described later in consideration of the efficient administration of the liposome by iontophoresis.

### Application of liposome formulation

The liposome formulation according to the present invention can be advantageously utilized in, for example, the prevention or therapy of a local dermatopathy, the application of an antioxidant component to the inside of a skin, and, furthermore, therapy including the extinction of active oxygen requiring a systemic action because the liposome formulation enables the stable, efficient delivery of the antioxidant component to each of the deep part of a pore and an intradermal tissue.

In addition, according to another aspect of the present invention, there is provided a method of administering an antioxidant component to a living organism by iontophoresis, the method including at least: placing the composition according to the present invention on the surface of the skin of the living organism; and energizing the above skin. In the above method, the antioxidant component encapsulated in the liposome of the formulation is administered to the living organism through a pore.

The liposome formulation according to the present invention may be placed on the surface of the skin by, for example, a method involving applying the liposome formulation as it is to the skin or a method involving causing an electrode assembly of an iontophoresis device to hold the formulation and placing the electrode assembly on the surface of the skin. The liposome in the present invention in which the antioxidant component is encapsulated may be energized as it is to be used in iontophoresis, and such aspect is also included in the present invention.

In the energization, a positive current is preferably applied on the anode side of an electrical system because the liposome in the present invention is cationic. A current value for the energization is preferably 0.1 to 0.6 mA/cm², more preferably 0.3 to 0.5 mA/cm², or still more preferably about 0.45 A/cm². In addition, the energization is performed for a time period of preferably 0.5 to 1.5 hours, more preferably 0.75 to 1.25 hours, or still more preferably about 1 hour.

In addition, the above living organism is preferably any one of the mammals, more preferably any one of a rat, a human being, a guinea pig, a rabbit, a mouse, and a pig, or still more preferably a human being.

### Electrode assembly and device for iontophoresis

As described above, the liposome formulation according to the present invention can be used by causing an electrode assembly for iontophoresis to hold the liposome formulation.

Therefore, according to a preferred aspect of the present invention, there is provided an electrode assembly for administering an antioxidant component to a living organism by iontophoresis, the electrode assembly holding the liposome formulation according to the present invention.

For example, in a preferred aspect in the present invention, the liposome to be used is cationic, and the electrode assembly is connected to the anode side of the electrical system of an iontophoresis device. Therefore, the electrode assembly according to the present invention includes at least a positive electrode and an antioxidant component holding portion held by the liposome formulation according to the present invention. In such electrode assembly, the above antioxidant component holding portion may be directly placed on the front surface of the positive electrode, and any other member such as an ion exchange membrane may be placed between the positive electrode and the antioxidant component holding portion as long as the member does not inhibit the administration of the liposome by iontophoresis. An example of the electrode assembly using such other member as described above is an electrode assembly including at least: a positive electrode; an electrolyte solution holding portion for holding an electrolyte solution, the electrolyte solution holding portion being placed on the front surface of the positive electrode; an anion exchange membrane placed on the front surface of the electrolyte solution holding portion; and an antioxidant component holding portion for holding the formulation according to the present invention. A cation exchange membrane may be further placed on the front surface of the above antioxidant component holding portion as desired.

Further, according to another aspect of the present invention, there is provided an iontophoresis device including the above electrode assembly. The iontophoresis device includes at least: a power supply; the electrode assembly holding the formulation according to the present invention, the electrode assembly being connected to the power supply; and an electrode assembly as a counter electrode of the electrode assembly. Although the constitution of the electrode assembly as a counter electrode is not particularly limited as long as the constitution does not inhibit the administration of the liposome by iontophoresis, the electrode assembly can be constituted of, for example, a negative electrode, an electrolyte solution holding portion for holding an electrolyte solution, the electrolyte solution holding portion being placed on the front surface of the negative electrode, and an ion exchange membrane placed on the front surface of the electrolyte solution holding portion. The above ion exchange membrane, which may be an anion exchange membrane or a cation exchange membrane, is preferably an anion exchange membrane.

Specific examples of the electrode assembly and the iontophoresis device according to the present invention as described above include those described in Fig. 1 to be described later and in International Patent WO 03/037425A1 by the applicant of the present invention.

In the iontophoresis device according to the present invention, the liposome migrates to the side opposite to the positive electrode by virtue of an electric field at the time of energization, and is efficiently released from the electrode assembly. Therefore, according to another aspect of the present invention, there is provided a method of activating the iontophoresis device, the method including: placing the electrode assembly according to the present invention and the electrode assembly as a counter electrode on the surface of the skin of a living organism; and energizing the iontophoresis device to release the liposome from the electrode assembly according to the present invention.

In the above iontophoresis device, the antioxidant component holding portion/the electrolyte solution holding portion may be constituted in the form of a cell (electrode chamber) made of, for example, acrylic and filled with the composition according to the present invention/the electrolyte solution, or may be constituted of a thin film body having property of holding the composition according to the present invention/the electrolyte solution by being impregnated with the composition/the electrolyte solution. Materials of the same kind can be used in the thin film bodies in the antioxidant component holding portion and the electrolyte solution holding portion.

In addition, a desired electrolyte solution can be appropriately used as the electrolyte solution depending on the characteristic conditions of the antioxidant component to be applied. However, an electrolyte solution that cause damage to the skin of a living organism owing to an electrode reaction should be avoided. An organic acid present in the metabolic cycle of a living organism, or a salt of the acid is a suitable electrolyte solution in the present invention from the viewpoint of harmlessness. For example, lactic acid or fumaric acid is preferable, and, specifically, an aqueous solution containing 1-M lactic acid and 1-M sodium fumarate at a ratio of 1 : 1 is particularly preferable.

In addition, it is important for the thin film body of which the antioxidant component holding portion is constituted to have a sufficient ability to hold the composition or the electrolyte solution by being impregnated with the composition or the electrolyte solution and a sufficient ability to transfer an ionized liposome held by the thin film body by being impregnated with the liposome under a predetermined electric field condition to a skin side (ion transferability or ion conductivity). A material that brings together good property of holding the composition by being impregnated with the composition and good ion transferability is, for example, a hydrogel body of an acrylic resin (acrylic hydrogel membrane), a segmented polyurethane-based gel membrane, or an ion conductive porous sheet for the formation of a gel-like solid electrolyte (for example, a porous polymer which: is disclosed in Japanese Patent Application Laid-open No. Sho 11-273452; and is based on an acrylonitrile copolymer containing 50 mol% or more, or preferably 70 to 98 mol% or more of acrylonitrile and having a porosity of 20 to 80%). In addition, when such antioxidant component holding portion as described above is impregnated with the composition, the degree of impregnation (100 x (W - D)/D [%] when the weight of the portion in a dry state is represented by D and the weight of the portion after the impregnation is represented by W) is preferably 30 to 40%.

Conditions under which the antioxidant component holding portion/the electrolyte solution holding portion is impregnated with the composition according to the present invention/the electrolyte solution are appropriately determined depending on, for example, the amount in which each of the portions is impregnated with the electrolyte solution or an ionic drug, and the rate at which each of the portions is impregnated with the electrolyte solution or the ionic drug. The impregnation is performed under, for example, the following conditions: 40°C for 30 minutes.

In addition, an inert electrode composed of, for example, a conductive material such as carbon or platinum can be preferably used as the electrode of the electrode assembly.

In addition, a cation exchange membrane and an anion exchange membrane are preferably used in combination as ion exchange membranes to be used in the electrode assembly. Preferable examples of the cation exchange membrane include NEOSEPTA's manufactured by Tokuyama Soda, Co., Inc. (CM-1, CM-2, CMX, CMS, CMB, and CLE 04-2). In addition, preferable examples of the anion exchange membrane include NEOSEPTA's manufactured by Tokuyama Soda, Co., Inc. (AM-1, AM-3, AMX, AHA, ACH, ACS, ALE 04-2, and AIP-21). Other preferable examples of the membranes include a cation exchange membrane obtained by partially or entirely filling the pore portions of a porous film with an ion exchange resin having a cation exchange function and an anion exchange membrane obtained by partially or entirely filling the pore portions of a porous film with an ion exchange resin having an anion exchange function.

Details about the respective components and the like described above are described in International Patent WO 03/037425A1 by the applicant of the present invention, and the contents described in the document are incorporated into the present invention.

### Example

### Example

### Preparation of liposome formulation

First, a liposome formulation for iontophoresis was prepared by encapsulating superoxide dismutase (SOD) as an active oxygen-extinguishing enzyme in a liposome (containing a cationic lipid DOTAP) with a stable lipid membrane composition capable of being used in iontophoresis by the following method.

250 µL of a solution of 10 mM of DOTAP (Avanti Polar Lipids, Inc.) in CHCl₃, 125 µL of a solution of 10 mM of cholesterol (hereinafter referred to as "Chol"; Avanti Polar Lipids, Inc.) in CHCl₃, and 250 µL of a solution of 10 mM of yolk phosphatidylcholine (NOF CORPORATION) in CHCl₃ were mixed. 500 µL of CHCl₃ were added to the resultant mixed liquid, whereby a suspension (molar ratio; DOTAP : Chol : yolk phosphatidylcholine = 2 : 1 : 2) was obtained. After the solvent of the suspension had been removed by distillation under reduced pressure with an evaporator, 400 µL of CHCl₃ were added to the remainder, and the solvent of the mixture was removed by distillation under reduced pressure again, whereby a lipid film was obtained. 1 mL of a 10-mM HEPES buffer and 0.5 mL of a solution of 5 mg (corresponding to 4,470 units/mg)/ml of SOD (manufactured by Sigma-Aldrich) in a 10-mM phosphate buffer (pH 7.4) were added to the lipid film. The resultant mixed liquid was left at room temperature for 10 minutes so as to be hydrated, and was then subjected to sonication (AU-25C ultrasonic cleaner manufactured by AIWA MEDICAL INDUSTRY CO., LTD., 85 W, room temperature, 1 minute). Further, the mixed liquid was treated with an extruder (product name: Mini-Extruder, manufactured by Avanti Polar Lipids, Inc.) by using PC membranes each having a pore size of 400 nm or 100 nm (product name: Nuclepre Track-Etch Membrane, manufactured by Whatman), whereby a liposome suspension was obtained. The resultant liposome suspension (liposome formulation) had an average particle diameter in the range of about 260 to 400 nm.

### Transdermal administration test

The liposome formulation obtained in the foregoing was transdermally administered to the back of a rat whose the back was shaved by iontophoresis with an iontophoresis device according to the following instruction.

First, anesthesia (1 mL of Nembutal (50 mg/ml) per 1 kg of a body weight) was administered to each of SD rats (male, 9 weeks old, manufactured by CLEA Japan, Inc.), and hair on the back of each rat was shaved. Next, as shown in Fig. 1, an iontophoresis device 1 constituted of a power supply 2, a working electrode assembly 3, and a non-working electrode assembly 4 was placed on an exposed skin 5. Here, 100 µL of the above liposome suspension were applied in advance to a surface where the exposed skin 5 and the working electrode assembly 3 contacted with each other. In addition, in the above iontophoresis device 1, the working electrode assembly 3 was constituted of: a positive electrode 31; an electrolyte solution holding portion 32 for holding 1 mL of an electrolyte solution (physiological saline), the electrolyte solution holding portion 32 being placed on the front surface of the positive electrode 31; an anion exchange membrane 33; and an antioxidant component holding portion 34 for holding 200 µL of the liposome suspension, the antioxidant component holding portion 34 being placed on the front surface of the anion exchange membrane 33. On the other hand, the non-working electrode assembly 4 was constituted of: a negative electrode 41; an electrolyte solution holding portion 42 for holding 1 mL of an electrolyte solution, the electrolyte solution holding portion 42 being placed on the front surface of the negative electrode 41; a cation exchange membrane 43; an electrolyte solution holding portion 44 for holding 800 µL of a physiological saline, the electrolyte solution holding portion 44 being placed on the front surface of the cation exchange membrane 43; and an anion exchange membrane 45 placed on the front surface of the electrolyte solution holding portion 44. In addition, ion exchange membranes stored in a physiological saline in advance were used as the above anion exchange membranes 33 and 45 (ALE 04-2 manufactured by Tokuyama Soda, Co., Inc.), and the cation exchange membrane 43 (CLE 04-2 manufactured by Tokuyama Soda, Co., Inc.).

Next, the liposome formulation was administered to each of some of the rats with the iontophoresis device 1 shown in Fig. 1 by energization under the following conditions: 1.14 mA (0.45 mA/cm²) for 1 hour.

1 hour after the administration of the liposome formulation, a dye (8-methoxypsoralen) capable of producing active oxygen by being irradiated with ultraviolet light was applied to each rat to which the liposome formulation had been administered. After that, each rat to which the liposome formulation had been administered was irradiated with ultraviolet light from a UVA (365 nm) lamp for 4 hours (34.5 J/cm²).

44 hours after the completion of the irradiation, the skin of each rat to which the liposome formulation had been administered was recovered, and the amount of a lipid peroxide (amount of malon dialdehyde MDA) in the skin was determined by a TBA method. Simultaneously with the determination, the skin was evaluated for oxidative damage by immunostaining with an antibody for detecting oxidative damage (an anti-MDA antibody (lipid oxidation), an anti-(hexanoyl)lysine antibody (protein oxidation), or anti-8-OH-deoxyguanosine (DNA oxidation)).

As a result, the skin of each rat irradiated with ultraviolet light underwent an inflammation, and a spot was observed on the surface of the skin. However, the skin of each rat to which the SOD-encapsulated liposome formulation had been administered was identical to that before the irradiation with ultraviolet light (see Figs. 2(A) and 2(B)). Fig. 2(A) shows the case where a skin was irradiated with ultraviolet light without the administration of the SOD-encapsulated liposome formulation, and, in this case, an inflammation is observed on the skin. On the other hand, Fig. 2(B) shows an example of the case where a skin was irradiated with ultraviolet light after the administration of the SOD-encapsulated liposome formulation, and, in this case, the skin maintained the same state as that before the irradiation with ultraviolet light.

Further, the skin of each rat irradiated with ultraviolet light was subjected to immunostaining. As a result, the skin was significantly stained by an antibody against oxidative damage, but the presence of oxidative damage was not observed in a rat to which the SOD-encapsulated liposome formulation had been administered. In addition, comparison between the MDA amount of a rat to which the SOD-encapsulated liposome formulation had been administered and the MDA amount of a rat to which no liposome formulation had been administered showed that a value for the former was lower than a value for the latter. By the way, such protecting effects on the skin of a rat against oxidative damage and an increase in MDA amount were not observed in the mere application of the SOD-encapsulated liposome formulation to the surface of the skin.

Figs. 3 and 4 show those results. A graph shown in Fig. 3 shows the result of the determination of the amount of a lipid peroxide (malon dialdehyde: MDA) of each of the skin of a rat to which the SOD-encapsulated liposome formulation had not been administered (UV) and the skin of a rat to which the SOD-encapsulated liposome formulation had been administered (SOD) after irradiation with UV. The amount of lipid peroxide is shown here as a molar amount per gram of skin (nmol/g wet weight).

On the other hand, Figs. 4(A) to 4(F) each show a photograph obtained by the fluorescence observation of a skin section of each of a rat to which the SOD-encapsulated liposome formulation had not been administered (UV) and a rat to which the SOD-encapsulated liposome formulation had been administered (SOD) after irradiation with UV, the section being subjected to immunostaining with each of various peroxidative damage marker antibodies, with a confocal laser microscope. That is, Fig. 4(A) shows the skin of a rat to which the SOD-encapsulated liposome formulation had not been administered (UV), the skin being subjected to immunostaining with anti-(hexanoyl)lysine (HEL). Similarly, Fig. 4(B) shows the skin of a rat to which the SOD-encapsulated liposome formulation had not been administered (UV), the skin being subjected to immunostaining with anti-malon dialdehyde (MDA). Similarly, Fig. 4(C) shows the skin of a rat to which the SOD-encapsulated liposome formulation had not been administered (UV), the skin being subjected to immunostaining with anti-8-OH-deoxyguanosine (8-OHdG). Similarly, Fig. 4(D) shows to the skin of a rat to which the SOD-encapsulated liposome formulation had been administered (UV), the skin being subjected to immunostaining with anti-(hexanoyl)lysine (HEL). Similarly, Fig. 4(E) shows the skin of a rat to which the SOD-encapsulated liposome formulation had been administered (UV), the skin being subjected to immunostaining with anti-malon dialdehyde (MDA). Similarly, Fig. 4(F) shows the skin of a rat to which the SOD-encapsulated liposome formulation had been administered (UV), the skin being subjected to immunostaining with anti-8-OH-deoxyguanosine (8-OHdG).

Those results confirmed that the liposome formulation according to the present invention diffused from a pore to the inside of a skin while maintaining its structure, and, furthermore, confirmed that the administration of an SOD-encapsulated liposome formulation to the inside of a skin by iontophoresis was able to prevent or suppress a dermatopathy in the skin due to ultraviolet light effectively.

## Claims

1. A liposome formulation for iontophoresis for administering an antioxidant component to a living organism by iontophoresis, wherein the antioxidant component is encapsulated in a liposome.

2. The liposome formulation for iontophoresis according to claim 1, wherein the antioxidant component is an antioxidant enzyme.

3. The liposome formulation for iontophoresis according to claim 2, wherein the antioxidant enzyme is superoxide dismutase (SOD).

4. The liposome formulation for iontophoresis according to claim 2, wherein the antioxidant enzyme is glutathione peroxydase (GSH-Px) and/of catalase.

5. The liposome formulation for iontophoresis according to claim 1, wherein the liposome formulation is composed of a combination of liposomes in each of which superoxide dismutase (SOD), glutathione peroxidase (GSH-Px), or catalase is encapsulated as the antioxidant component.

6. The liposome formulation for iontophoresis according to any one of claims 1 to 5, wherein the liposome contains, as constituents, a cationic lipid, and an amphiphilic glycerophospholipid containing both a saturated fatty acid and an unsaturated fatty acid as constituent fatty acids.

7. The liposome formulation for iontophoresis according to claim 1, wherein the liposome formulation is used for prevention or therapy of a dermatopathy resulting from ultraviolet light.

8. The liposome formulation for iontophoresis according to claim 1, wherein the liposome formulation is used for administering the active ingredient to the living organism through a pore.

9. The liposome formulation for iontophoresis according to claim 6, wherein the cationic lipid is a C1 to C20 alkane substituted by a C1 to C22 acyloxy group and a tri(C1 to C6)alkylammonium group.

10. The liposome formulation for iontophoresis according to claim 9, wherein the number of the C1 to C22 acyloxy group in the cation lipid is 2, and the number of the tri(C1 to C6)alkylammonium group in the cation lipid is 1.

11. The liposome formulation for iontophoresis according to claim 6, wherein the cationic lipid is 1,2-dioleoyloxy-3-(trimethylammonium)propane.

12. The liposome formulation for iontophoresis according to claim 6, wherein the amphiphilic glycerophospholipid is phosphatidylcholine.

13. The liposome formulation for iontophoresis according to claim 6, wherein the amphiphilic glycerophospholipid is a yolk phosphatidylcholine.

14. The liposome formulation for iontophoresis according to claim 6, wherein the saturated fatty acid is a C12 to C22 saturated fatty acid.

15. The liposome formulation for iontophoresis according to claim 6, wherein the saturated fatty acid comprises at least one selected from the group consisting of palmitic acid, lauric acid, myristic acid, pentadecylic acid, margaric acid, stearic acid, tuberculostearic acid, arachidic acid, and behenic acid.

16. The liposome formulation for iontophoresis according to claim 6, wherein the unsaturated fatty acid contains 1 to 6 carbon-carbon unsaturated double bonds.

17. The liposome formulation for iontophoresis according to claim 6, wherein the unsaturated fatty acid is a C14 to C22 unsaturated fatty acid.

18. The liposome formulation for iontophoresis according to claim 6, wherein the unsaturated fatty acid comprises at least one selected from the group consisting of oleic acid, myristoleic acid, palmitoleic acid, elaidic acid, vaccenic acid, gadoleic acid, erucic acid, nervonic acid, linoleic acid, α-linolenic acid, eleostearic acid, stearidonic acid, arachidonic acid, eicosapentaenoic acid, clupanodonic acid, and docosahexaenoic acid.

19. The liposome formulation for iontophoresis according to claim 6, wherein the liposome further comprises a sterol as a constituent.

20. The liposome formulation for iontophoresis according to claim 19, wherein the sterols comprise at least one selected from the group consisting of cholesterol, a C12 to C31 fatty acid cholesteryl, a C12 to C31 fatty acid dihydrocholesteryl, polyoxyethylene cholesteryl ether, and polyoxyethylene dihydrocholesteryl ether.

21. The liposome formulation for iontophoresis according to claim 19, wherein the sterols comprise at least one selected from the group consisting of cholesterol, cholesteryl lanoate, cholesteryl oleate, cholesteryl nonanoate, and polyoxyethylene dihydrocholesteryl ether.

22. The liposome formulation for iontophoresis according to claim 19, wherein the sterols is cholesterol.

23. The liposome formulation for iontophoresis according to claim 19, wherein the cationic lipid is 1,2-dioleoyloxy-3-trimethylammonium propane, the amphiphilic glycerophospholipid is yolk phosphatidylcholine, and the sterol is cholesterol.

24. The liposome formulation for iontophoresis according to claim 6, wherein a molar ratio between the cationic lipid and the amphiphilic glycerophospholipid is 3 : 7 to 7 : 3.

25. The liposome formulation for iontophoresis according to claim 19, wherein a molar ratio between the cationic lipid and the sterol is 3 : 7 to 7 : 3.

26. The liposome formulation for iontophoresis according to claim 19, wherein a molar ratio between the amphiphilic glycerophospholipid and the sterols is 3 : 7 to 7 : 3.

27. The liposome formulation for iontophoresis according to claim 19, wherein a molar ratio between the cationic lipid and a sum of the amphiphilic glycerophospholipid and the sterols is 3 : 7 to 7 : 3.

28. The liposome formulation for iontophoresis according to claim 19, wherein a molar ratio between the cationic lipid, the amphiphilic glycerophospholipid, and the sterols is about 2 : 1: 1.

29. The liposome formulation for iontophoresis according to claim 1, wherein the liposome has an average particle diameter of 400 or more.

30. The liposome formulation for iontophoresis according to claim 1, wherein the liposome has an average particle diameter of 400 to 1,000 nm.

31. The liposome formulation for iontophoresis according to claim 1, wherein the liposome is administered to the living organism by iontophoresis at a current value of 0.1 to 0.6 mA/cm².

32. An electrode assembly for administering an antioxidant component to a living organism by iontophoresis, comprising the liposome formulation according to claim 1.

33. An iontophoresis device, comprising the electrode assembly according to claim 32.
